# EUROPÄISCHE PATENTSCHRIFT

(11) **EP 0 838 155 B1**
(45) Veröffentlichungstag und Bekanntmachung des Hinweises auf die Patenterteilung: **15.09.2004**
(21) Anmeldenummer: 97118052.6
(22) Anmeldetag: 17.10.1997
(51) Int. Cl.: A01N 45/00, A61K 7/32, A61K 7/48, A61K 7/06, A61K 31/575

(54) **Antiadhäsive Sterole und Sterolderivate**
Anit-adhesive steroids and steroid derivatives
Stéroides et leurs dérivés possédant des propriétés anti-adhésives

(30) Priorität: 22.10.1996 DE 19643587
(43) Veröffentlichungstag der Anmeldung: 29.04.1998
(73) Patentinhaber: Beiersdorf Aktiengesellschaft, 20245 Hamburg (DE)
(72) Erfinder: Bünger, Joachim, Dr., 22459 Hamburg (DE); Hoppe, Udo, Dr., 22397 Hamburg (DE); Wolf, Florian, Dr., 20251 Hamburg (DE)

(56) Entgegenhaltungen:
- WO-A-91/08745
- WO-A-96/23479
- WO-A-98/17241
- US-A- 4 719 101
- DATABASE WPI Week 199619, Derwent Publications Ltd., London, GB; AN 1996-184637, XP002053087 'Antibacterial agent preparation add vitanim organic solvent' & JP 8 059 407 A (KIKOMAN CORP) 05 M{rz 1996
- DATABASE WPI Week 197449, Derwent Publications Ltd., London, GB; AN 1974-85411V, XP002053088 'Purification Phytosterol emulsion base ointment cosmetic cream stabilised wide temperature range' & SU 404 478 A (LENINGRAD KIROV WOODTECH) 12 Juni 1974
- CHEMICAL ABSTRACTS, vol. 113, no. 25, 17.Dezember 1990 Columbus, Ohio, US; abstract no. 225189v, KAI ET AL.: "Antimicrobial compositions containing steroids" XP002053086 & JP 02 124 826 A
- DATABASE WPI Week 198043, Derwent Publications Ltd., London, GB; AN 1980-75881C, XP002053089 'Deodorant cleansing compsn. for removing oil from hands etc. -obtd. by mixing N-allyl-aminoacid esp. N-acyl-glutamic acid salt with powdered zeolite and moulding' & JP 55 116 798 A (AJINOMOTO KK) 08 September 1980

## Beschreibung

Gegenstand der Erfindung sind Verwendungen von Sterolen und Sterolderivaten und Zubereitungen, die diese Stoffe enthalten.

Es ist bereits bekannt, Mikroorganismen auf Oberflächen durch Mikrobizide abzutöten oder mit Reinigungsmitteln abzuspülen, um auf diese Weise ihre Anzahl auf der Fläche zu vermindern. Beide Methoden haben bekannte Nachteile. So können z.B. Desinfektionsmittel die Oberflächen schädigen und Reinigungsmittel reichen oft in der Wirkung nicht aus.

Aufgabe der Erfindung war es daher, eine schonende und wirkungsvolle Methode zu schaffen, mit der es gelingt, die Anzahl von grampositiven Bakterien auf Oberflächen gering zu halten oder zu verringern oder zu verhindern, daß grampositive Bakterien an Oberflächen haften.

Gegenstand der Erfindung ist die Verwendung einer Verbindung oder mehrerer Verbindungen aus der Gruppe der Sterole und Sterolderivate als antiadhäsive Wirkstoffe, insbesondere gegenüber grampositiven Bakterien.

Mit den Begriffen "antiadhäsiv" und "antiadhäsiver Wirkung" der erfindungsgemäßen Wirkstoffe ist gemeint, daß die Adhäsion von grampositiven Bakterien an Oberflächen herabgesetzt oder aufgehoben ist.

Die erfindungsgemäßen antiadhäsiven Wirkstoffe und Zubereitungen bewirken, daß sich auf Oberflächen nur noch geringe und keine störenden Ansammlungen von grampositiven Bakterien ausbilden oder sie verdrängen auch bereits anhaftende grampositive Bakterien von der Oberfläche und verringern so deren Anzahl. Sie können auch prophylaktisch verwendet werden.

Die genannten Aufgaben werden erfindungsgemäß gelöst. Die erfindungsgemäßen Wirkstoffe haben die erfindungsgemäßen, genannten Wirkungen auf grampositive Bakterien und sind zur Behandlung der genannten Störungen und Krankheiten geeignet. Die Patentschrift US 4,719,101 beschreibt Deodorantzubereitungen mit dem Wirkstoff Methylsalicylat. Die WO 91/08745 beschreibt die antiadhäsive Wirkung eines Sterolderivates gegenüber Viren. Beide Schriften konnten jedoch nicht den Weg zur vorliegenden Erfindung weisen.

Geeignete Sterole und Sterolderivate sind beispielsweise:
A) natürliche, natur-identische und synthetisch zugängliche Sterole
B) natürliche und synthetische Steroidhormone mit einer Sterol-Struktur, und
C) Naturstoffe und Naturstoffgemische, die Verbindungen mit einer Sterol-Struktur enthalten.

Gut geeignete Sterole (auch als Sterine bezeichnet) A) sind z.B. Zoosterole, Phytosterole und Mykosterole.

Bevorzugte Zoosterole sind Cholesterol, Dihydrocholesterol, 7-Dehydrocholesterol, Lanosterol, Dihydrolanosterol, Spongosterol und Stellasterol.

Bevorzugte Phytosterole sind Ergosterol, Sitosterol, Stigmasterol, Fucosterol, Brassicasterol und Campesterol.

Bevorzugte Mykosterole sind Ergosterol, Fungisterol und Zymosterol.

Bevorzugte Verbindungen sind insbesondere Cholsterol, 7-Dehydrocholesterol, Dihydrocholesterol, Lanosterol, Dihydrolanosterol, Spongosterol, Koprosterol, Phytosterole, Cholesteryloleat und Cholesterylsulfat.

Gut geeignet sind weiterhin Cholecalciferol, Ergocalciferol und Calciferol.

Geeignete Steroidhormone B) sind beispielsweise Androgene, Östrogene, Gestagene, Corticoide, Mineralcorticoide und Ecdyson.

Bevorzugte Naturstoffe und Naturstoffgemische C) sind Wollwachs und Wollwachsalkohole, Sojasterole, Tallölsterole und Naturstoffe mit Steroidgerüsten, beispielsweise:
- Cardenolide (z.B. Digoxygenin, Digitoxin, Digoxin, Gitoxin, Gitoxygenin, Digitoxygenin),
- Bufadienolide (z.B. Scillarenin, Scillaren),
- Steroid-Sapogenine (z.B. Digitogenin, Gitogenin, Tigogenin),
- Gallensäuren und deren Derivate (z.B. Cholsäure, Glykocholsäure, Taurocholsäure, Desoxycholsäure, Lithocholsäure, Chenodesoxycholsäure, Cholansäure), und
Steroid-Alkaloide (z.B. Solanidin, Tomatidin, Strophanitn, Conessin).

Gegenstand der Erfindung sind auch die hydrierten Derivate der Sterole der Gruppen A, B und C, insbesondere der Gruppe A.

Sie können z.B. in der 5/6-Stellung, 7/8-Stellung und/oder 8/9-Stellung hydriert sein.

Bevorzugt werden die Dihydroderivate, insbesondere die 5/6-Dihydroderivate.

Weiterhin sind auch die Dehydroderivate der Sterole der Guppen A, B und C geeignet, insbesondere die 7-Dehydroderivate.

Bevorzugt werden Dihydrocholesterol, Dihydrolanosterol und 7-Dehydrocholesterol.

Geeignete Derivate der Verbindungen der Gruppen A, B und C, die auch in den hydrierten Formen, insbesondere Dihydroformen, oder den Dehydroformen vorliegen können, sind auch solche Derivate, die durch Substitution der 3-Hydroxygruppe der Sterole erhältlich sind und im folgenden auch mit D bezeichnet werden. Bevorzugt werden diese Derivate von Verbindungen der Gruppe A.

Bevorzugt werden diese 3-OH-Gruppen mit anorganischen oder organischen Säuren oder mit Alkoholen umgesetzt, also verestert oder verethert, oder aber mit Aminen umgesetzt, wobei sich durch Ersatz der Sterol-3-OH-Gruppe eine substituierte 3-Amino-Gruppe des Sterol-Grundgerüstes ergibt.
a) Die Sterol-3-OH-Gruppe kann beispielsweise mit anorganischen Säuren wie Schwefelsäure oder Phosphorsäure verestert werden. Bevorzugt werden die Sulfate, insbesondere die Sulfate der Verbindungen der Gruppe A, z.B. Cholesteryl-3-sulfat.
b) Die 3-OH-Gruppe kann mit Carbonsäuren verestert sein. Geeignet sind z.B. verzweigte oder unverzweigte, gesättigte oder ungesättigte, vorzugsweise ein- bis 5-fach, insbesondere ein- oder zweifach ungesättigte Carbonsäuren mit 1 - 40, vorzugsweise 1 - 20 Kohlenstoffatomen.
   Bevorzugt werden Cholesterylformiat, Cholesterylisostearat, Cholesteryloleat und beta-Sitosterylacetat.
   Besonders bevorzugt werden die genannten Carbonsäureester des Sojasteroles, insbesondere Sojasterolacetat.
   Weiterhin werden die genannten Carbonsäureester der Phytosterole und Dihydrophytosterole bevorzugt, insbesondere die Octadecanoate, z.B. Dihydrophytosteryloctadecanoat.
c) Die 3-OH-Gruppe kann auch mit hydroxylierten Carbonsäuren verestert sein. Geeignet sind z.B. ein- bis fünffach hydroxylierte Carbonsäuren, z.B. wie unter b) beschrieben. Mono- und Dihydroxycarbonsäuren werden bevorzugt.
   Bevorzugt werden Cholesterylhydroxystearate, vorzugsweise die Monohydroxystearate, vorzugsweise Cholesteryl-α-hydroxystearat.
d) Die 3-OH-Gruppe kann auch mit Alkoholen verethert vorliegen. Geeignet sind z.B. geradkettige oder verzweigte, gesättigte oder ein- bis fünffach, vorzugsweise ein- oder zweifach ungesättigte Monoalkohole mit 1 - 40, insbesondere 1 - 20 Kohlenstoffatomen.
e) Die 3-OH-Gruppe kann auch mit Polyolen verethert sein. Geeignet sind geradkettige oder verzweigte, gesättigte oder ein- bis fünffach, vorzugsweise ein- oder zweifach ungesättigte Polyhydroxyalkanole mit 2 - 8, vorzugsweise 2 - 6, insbesondere 2 oder 3 Hydroxygruppen.
   Bevorzugt werden Glyceryllanolat und die mit Sorbit, Mannit oder Xylit erhältlichen Sterolether, insbesondere die des Cholesterols.
f) Die 3-OH-Gruppe kann auch mit Cycloalkylgruppen, die gegebenenfalls mit einer oder zwei oder mehreren, Hydroxygruppen substituiert sein können, verethert sein.
   Die Cycloalkylgruppen können 4 - 8, insbesondere 6 Kohlenstoffatome besitzen und ggf. an jedem C-Atom eine Hydroxygruppe tragen.
g) Die 3-OH-Gruppe kann weiterhin mit glycosidisch gebundenen Zuckerresten substituiert sein, beispielsweise Mono-, Di- oder Oligosacchariden mit vorzugsweise 2 oder 3 Zuckerresten. Geeignete Saccharide, insbesondere Monosaccharide, sind Triosen, Tetrosen, Pentosen oder Hexosen, davon insbesondere die Pentosen und Hexosen. Glucose und Fruktose werden besonders bevorzugt. Insbesondere wird Cholesterylglucosid bevorzugt.
h) Geeignete 3-OH-Derivate sind weiterhin die Ethoxylate, z.B. solche mit Ethylenoxid-Ketten mit einer E/O-Zahl von 1 - 200, insbesondere 5 - 150. Bevorzugt werden Choleth-20, Dihydrocholet-30, Laneth-5 bis -50, PEG-50-Lanolin bis PEG-150-Lanolin, PEG-5-Sojasterol bis PEG-50 Sojasterol, PEG-5-Tallölsterolether.
i) Geeignete 3-OH-Derivate sind weiterhin die Propoxylate, z.B. solche mit einer P/O-Zahl von 1 - 200, insbesondere 5 - 30. Bevorzugt werden PPG-5-Lanolin-Wachs, PPG-5-Lanonlin-Alkohol-Ether bis PPG-30-Lanolin-Alkohol-Ether.
j) Geeignet sind weiterhin die gemischten Ethoxylate-Propoxylate der 3-OH-Gruppe. Geeignete Ethoxylat- oder Propoxylat-Reste können jeweils 1 - 100, vorzugsweise 10 - 100 dieser Reste besitzen. Bevorzugte gemischte (E/O)ₙ-(P/O)ₘ-Ketten mit den vorstehend angegebenen Zahlenwerten für n und m sind PPG-12-PEG-50-Lanolin und PPG-40-PEG-60-Lanolin-Öl.
k) Bevorzugte, an der 3-OH-Gruppe substituierte Sterole sind auch die Sulfosuccinate. Bevorzugt wird Di-Natrium-Sitostereth-14-Sulfosuccinat.
l) Weiterhin werden die Umsetzungsprodukte der 3-OH-Gruppe der Sterole, insbesondere des Cholesterols, mit den Aminogruppen von Aminosäuren bevorzugt, wobei z.B. in einer Kondensationsreaktion unter Wasserabspaltung die 3-OH-Gruppe durch den Amino-Rest der Aminosäure ersetzt wird, wodurch die 3-Amino-Derivate des C-3-Atomes des Sterol-Ringsystems gebildet werden. Besonders bevorzugt wird das in dieser Weise erhältliche Amin, das durch Umsetzung von Glutaminsäure mit Cholesterol erhältlich ist.

Besonders bevorzugt werden die vorstehend unter a) bis l) beschriebenden Derivate D des Cholesterols.

Besonders bevorzugt werden die folgenden erfindungsgemäßen Sterole und Sterolderivate:

| Substanz | Handelsname | Lieferant/Hersteller |
|---|---|---|
| | | |
| Cholesteryl-Sulfat | | |
| Beta-Sitosterol | | |
| Beta-Sitosterylacetat | Sitostearyl Complex | Variati |
| Cholecalciferol | Epiderm-Complex O | Cosmetochem |
| Cholesterol | Cholesterol USP/NF | Croda |
| Cholesteryl Hydroxystearat | Estemol CHS | beide Nissin |
| | Salacos HS | Oil Mills |
| Cholesteryl lsostearat | IS-CE | Kao Corp. |
| Choleth-20 | Nikkol Aquasome EC-5 | Nikko |
| Choleth-24 | Fancol CH-24 | Fanning |
| Dihydrocholesterol | | |
| Dihydrocholesteryl Octyldecanoat | Nikkol DCIS | Nikko |
| Dihydrocholeth-15 | Nikkol DHC-15 | Nikko |
| Dihydrocholeth-20 | Nikkol DHC-20 | Nikko |
| Dihydrocholeth-30 | Nikkol DHC-30 | Nikko |
| Dihydrolanosterol | Isocholesterol EX | Nikko |
| Dihydrophytosteryl Octyldecanoat | Nikkol DPIS | Nikko |
| Disodium Sitostereth-14 Sulfosuccinat | Rewoderm SPS | Rewo Chem. |
| Ergocalciferol | | |
| Glyceryl Lanolat | Ivarlan 3360 | Brooks |
| | Neocerit | BDF |
| Laneth-5 | Polychol-5 | Croda |
| Laneth-10 | Polychol-10 | Croda |
| Laneth-15 | Polychol-15 | Croda |
| Laneth-20 | Polychol-20 | Croda |
| Laneth-25 | Soluan 25 | Amerchol |
| Laneth-50 | Sterol LN 50 | Auschem |
| Laneth-10 Acetat | Lipolan 98 | Lipo |
| Lanosterol | Lanosterol | Croda |
| | Isocholesterol EX | Nikko |
| PEG-5-Lanolat | Agnosol 5 | Croda |
| PEG-10-Lanolat | Sklirate 10 | Croda |
| PEG-20-Lanolat | Sklirate 20 | Croda |
| PEG-10-Lanolin | Nikkol TW-10 | Nikko |
| PEG-20-Lanolin | Solan 20 | Croda |
| PEG-30-Lanolin | Nikkol TW-30 | Nikko |
| PEG-40-Lanolin | Sol'an 40 | Croda |
| PEG-75-Lanolin | Aqualose L75 | Westbrook Lanolin |
| PEG-100-Lanolin | Aqualose L100 | Westbrook Lanolin |
| PEG-150-Lanolin | Solan X | Croda |
| PEG-25-Phytosterol | | |
| PEG-5-Soya Sterol | | |
| PEG-10-Soya Sterol | | |
| PEG-25-Soya Sterol | | |
| PEG-40-Soya Sterol | | |
| PEG-5 Tall Oil Sterol Ether | | |
| PPG-2 Lanolin Alcohol Ether | Soluan PB-2 | Amerchol |
| PPG-5-Lanolin Alcohol Ether | Soluan PB-5 | Amerchol |
| PPG-10-Lanolin Alcohol Ether | Soluan PB-10 | Amerchol |
| PPG-20-Lanolin Alcohol Ether | Soluan PB-20 | Amerchol |
| PPG-30-Lanolin Alcohol Ether | Soluan PB-30 | Amerchol |
| PPG-5-Lanolin Wax | Propoxyol 1695 | Henkel |
| PPG-12-PEG-50 Lanolin | Lanexol AWS | Croda |
| PPG-12-PEG-65 Lanolin Oil | Fluilan AWS | Croda |
| PPG-40-PEG-60 Lanolin Oil | Aqualose LL 100 | Westbrook L. |
| Soy Sterol | | |
| Soy Sterol Acetate | | |
| Tall Oil Sterol | | |
| C10 -C30 Cholesterol/Lanosterol Esters | | |

Die Aufbereitung der erfindungsgemäßen Wirkstoffe erfolgt nach dem Fachmann geläufigen, üblichen Methoden.

Erfindungsgemäße Wirkstoffe sind im Handel erhältlich oder bekannt oder können nach bekannten Verfahren erhalten werden.

Zubereitungen, insbesondere topische Zubereitungen, z.B. kosmetische und dermatologische Zubereitungen, mit den erfindungsgemäßen Wirkstoffen können diese z.B. in Mengen von 0,01 bis 25,0 Gew.-%, vorzugsweise 5,0 bis 20,0 Gew.-%, insbesondere aber 2,0 bis 10 Gew.-%, jeweils bezogen auf das Gesamtgewicht der Zubereitungen, enthalten. Insbesondere gelten diese Mengen auch jeweils für die Einzelkomponenten der Kombinationen.

Überraschenderweise wurde gefunden, daß die erfindungsgemäßen Wirkstoffe und Desodorantien, die sie enthalten, die Adhäsion, d.h. das Vermögen z.B. der grampositiven Bakterien an Oberflächen anzuhaften, herabsetzen, so daß sich deren übliche Anzahl auf solche Flächen verringert, oder auch, daß sich keine oder keine wesentlichen Mengen von grampositiven Bakterien mehr nachweisen lassen.

Solche Oberflächen sind z.B. Organaußenflächen oder Organ-Oberflächen, insbesondere von der Haut oder Schleimhaut und Körperhöhlen oder Organhohlräume.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff Antibiotika beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive Bakterien antiadhäsiv wirksam sind, ohne daß mit der Anwendung der Substanzen eine unvertretbare Beeinträchtigung der Gesundheit des Anwenders verbunden wäre.

Grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw..

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der grampositiven Bakterien aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Durch die erfindungsgemäßen Wirkstoffe in Desodorantien werden die vorstehenden Aufgaben gelöst. Die erfindungsgemäßen Wirkstoffe eignen sich hervorragend zur Behandlung der genannten Zustände.

Mit den erfindungsgemäßen Wirkstoffen und Zubereitungen, die diese enthalten, kann die Adhäsion aller grampositiven Bakterien an Oberflächen verringert oder vermieden werden.

Insbesondere die folgenden grampositiven Bakterien und die durch sie hervorgerufenen Störungen und Krankheiten können erfindungsgemäß besonders gut topisch behandelt werden:

### 1. Grampositive Bakterien

Als Beispiele:
Fakultativ pathogene und pathogene Micrococcaceae, insbesondere Staphylococcus epidermidis, z.B. bei der Entstehung von Achselgeruch und beim atopischen Ekzem sowie Staphylococcus aureus als wichtiges Pathogen, z.B. beim atopischen Ekzem, bei Neurodermitis und Psoriasis, Corynebacterium spec., z.B. bei der Entstehung des Achselgeruches, Propionibacterium spec., z.B. bei der Entstehung von Akne und bei unreiner Haut.

Die Anwendung der Wirkstoffe kann topisch erfolgen.

Zubereitungen, die erfindungsgemäße Wirkstoffe enthalten, können topische Zubereitungen sein, beispielsweise kosmetische und dermatologische topische Zubereitungen. Bevorzugt werden Desodorantien oder desodorierende Körperreinigungsprodukte oder Körperpflegeprodukte. Die Wirkstoffe können aber auch in Desinfektionsmitteln und/oder Reinigungsmitteln enthalten sein, die nicht nur zur Behandlung des Körpers oder der Haut bestimmt sind, sondern auch zum Reinigen und Desinfizieren von harten Oberflächen, medizinischen Materialien, Geräten, Instrumenten, Mobiliar und Wänden.

Für den Körper bestimmte Reinigungsmittel, Desinfektionsmittel, Spülmittel können ebenfalls zur Behandlung von der Haut verwendet werden wie schon die topischen Zubereitungen. Sie dienen aber vorzugsweise zur Desodorierung von Körperhöhlen wie auch des Mund- und Rachenraumes sowie der Nase.

Die Wirkstoffe gemäß der Erfindung können mit üblichen pharmazeutisch verträglichen Verdünnungsmitteln oder Trägern und gegebenenfalls mit anderen Hilfsmitteln vermischt und verabreicht werden. Sie können in Form von Granulaten, Pillen, Tabletten, Dragees, Sirupen, Emulsionen, Suspensionen, Dispersionen, Aerosolen und Lösungen sowie Flüssigkeiten, oder in Form von Lösungen, Emulsionen oder Suspensionen verabreicht werden.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können. Die Applikation erfolgt in üblicher Weise, vorzugsweise oral, insbesondere perlingual oder topisch. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die Desodorantien können den Wirkstoff in einer Menge von 0,1 bis 90 Gewichtsprozent, insbesondere 1 - 90 Gew.-% enthalten. Einzeldosen enthalten die Wirkstoffe vorzugsweise in einer Menge von 0,1 bis 10 g.

Die erfindungsgemäßen desodorierenden Formulierungen können wie üblich zusammengesetzt sein und zur Behandlung der Haut und/oder der Haare im Sinne einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind auch solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Die Zusätze der UVA-Filter und UVB-Filter oder Pigmente sind auch zur Stabilisierung der Zubereitungen geeignet.

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können als Sonnenschutzmittel dienen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)-campher,
- 4-Aminobenzoesäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Kosmetische und dermatologische Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung, eine Emulsion vom Typ Wasserin-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. ∝-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. ∝-Hydroxyfettsäuren, Palmitinsäure, Phytinsäure, Lactoferrin), ∝-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ -Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrigalkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwassertoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine erfindungsgemäße ethoxylierte oder propoxylierte organische Verbindung im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenative Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben.

Kosmetische und dermatologische Zubereitungen zur Behandlung und Pflege der Haare können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben mindestens einem erfindungsgemäßen alkylierten Hydrochinon und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Soweit nicht anders angegeben, beziehen sich Mengenangaben, Prozentangaben und Teile auf das Gewicht, insbesondere auf das Gesamtgewicht der jeweiligen Mischung oder Zubereitung.

Gegenstand der Erfindung ist auch die Verwendung der erfindungsgemäßen Wirkstoffe zur Herstellung von Zubereitungen, insbesondere pharmazeutischen Zubereitungen zur Behandlung der angegebenen Störungen oder Krankheiten.

Die folgenden Beispiele sollen die Verkörperungen der vorliegenden Erfindungen verdeutlichen. Die angegebenen Zahlenwerte sind Gew.-%.

### Beispiel 1

| W/O-Crème | |
|---|---|
| Paraffinöl | 10,00 |
| Ozokerit | 4,00 |
| Vaseline | 4,00 |
| pflanzliches Öl | 10,00 |
| Wollwachsalkohol | 2,00 |
| Aluminiumstearat | 0,40 |
| Dihydrocholesteryl-octyldecanoat | 5,00 |
| Parfum, Konservierungsstoffe | ................... q.s. ................... |
| Wasser, VES | .............. ad 100,00 .............. |

Das o.g. Beispiel kann auch mit einer identischen Menge Glyceryllanolat, Cholesteryl-isostearat oder Laneth-10 anstelle von Dihydrocholesteryl-octyldecanoat formuliert werden.

### Beispiel 2

| W/O-Lotion | |
|---|---|
| Paraffinöl | 25,00 |
| Siliconöl | 2,00 |
| Ceresin | 1,50 |
| Wollwachsalkohol | 0,50 |
| Glucosesesquiisostearat | 2,50 |
| Dihydrophytosteryl-oclyldecanoat | 7,00 |
| Parfum, Konservierungsstoffe | ................... q.s. ................... |
| Wasser, VES | .............. ad 100,00 .............. |

Das o.g. Beispiel kann auch mit einer identischen Menge Dihydrocholet-5, Cholesterol oder Cholecalciferol anstelle von Dihydrophytosteryl-octyldecanoat formuliert werden.

### Beispiel 3

| O/W-Lotion | |
|---|---|
| Paraffinöl | 5,00 |
| Isopropylpalmitat | 5,00 |
| Cetylalkohol | 2,00 |
| Bienenwachs | 2,00 |
| Ceteareth-20 | 2,00 |
| PEG-20-Glycerylstearat | 1,50 |
| Glycerin | 3,00 |
| Lanosterol | 2,00 |
| Parfum, Konservierungsstoffe | ................... q.s. ................... |
| Wasser, VES | .............. ad 100,00 .............. |

Das o.g. Beispiel kann auch mit einer identischen Mengen PPG-5-Lanolin Wax, PEG-25-Phytosterol oder PEG-40 Soya Sterol anstelle von PEG-20-Glycerylstearat formuliert werden.

### Beispiel 4

| O/W-Crème | |
|---|---|
| Pflanzliches Öl | 10,00 |
| Cetylalkohol | 2,00 |
| Glycerinmonostearat | 1,50 |
| PEG-30-Glycerylstearat | 2,00 |
| Glycerin | 3,00 |
| Isopropylpalmitat | 5,00 |
| Carbopol 980 (neutralisiert) | 0,30 |
| PPG-10-Lanolin Alcohol Ether | 2,00 |
| Parfum, Konservierungsstoffe | ................... q.s. ................... |
| Wasser, VES | .............. ad 100,00 .............. |

Das o.g. Beispiel kann auch mit einer identischen Menge an PEG-10 Lanolin, PEG-20 Soya Sterol oder Tall Oil Sterol anstelle von PPG-10-Lanolin Alcohol Ether formuliert werden.

### Beispiel 5

| Salbe | |
|---|---|
| Vaseline | 36,00 |
| Ceresin | 10,00 |
| Zinkoxid | 4,00 |
| Pflanzliches Öl | 20,00 |
| Wollwachsalkohol | 3,50 |
| Parfum, Konservierungsstoffe | ................... q.s. ................... |
| Paraffinöl | .............. ad 100,00 .............. |

Das o.g. Beispiel kann auch mit einer identischen Menge an Lanosterol, PEG-25-Phytosterol oder Cholesteryloleat anstelle von Wollwachsalkohol formuliert werden.

### Beispiel 6

| Hautöl | |
|---|---|
| Cetylpalmitat | 3,00 |
| C₁₂₋₁₅-Alkylbenzoat | 2,00 |
| Polyisobuten | 10,00 |
| Squalan | 2,00 |
| β-Sitosterol | 5,00 |
| Parfum, Konservierungsstoffe | ................... q.s. ................... |
| Paraffinöl | .............. ad 100,00 .............. |

Das o.g. Beispiel kann auch mit einer identischen Menge an Dihydrocholesterol, Disodium Sitostereth-14-sulfosuccinat oder PEG-10-Soya Sterol anstelle von β-Sitosterol formuliert werden.

### Beispiel 7

| Badeöl | |
|---|---|
| Paraffinöl | 20,00 |
| PEG-40-hydriertes Rizinusöl | 5,00 |
| Cholesteryl-Hydroxystearat | 5,00 |
| Parfum, Konservierungsstoffe | ................... q.s. ................... |
| Sojaöl | .............. ad 100,00 .............. |

Das o.g. Beispiel kann auch mit einer identischen Menge an Laneth-20, PPG-5 Lanolin Wax oder PEG-25 Phytosterol anstelle von Cholesteryl-Hydroxystearat formuliert werden.

### Beispiel 8

| Lippenstift | |
|---|---|
| Ceresin | 8,00 |
| Bienenwachs | 4,00 |
| Carnaubawachs | 2,00 |
| Vaseline | 40,00 |
| Hydriertes Rizinusöl | 4,00 |
| Cholesterol | 2,00 |
| Parfum, Konservierungsstoffe | ................... q.s. ................... |
| Paraffinöl | .............. ad 100,00 .............. |

Das o.g. Beispiel kann auch mit einer identischen Menge an Cholesteryloleat, Lanosterol oder Soya Sterol anstelle von Cholesterol formuliert werden.

### Beispiel 9

| Pflegemaske | |
|---|---|
| PEG-50 Lanolin | 0,50 |
| Glycerylstearat | 2,00 |
| Sonnenblumenkernöl | 3,00 |
| Bentonit | 8,00 |
| Kaolin | 35,00 |
| Zinkoxid | 5,00 |
| PEG-5-Phytosterol | 1,00 |
| Parfum, Konservierungsstoffe | ................... q.s. ................... |
| Wasser, VES | .............. ad 100,00 .............. |

Das o.g. Beispiel kann auch mit einer identischen Menge an PEG-5-Lanolat, PPG-5-Lanolin W ax o der Soya Sterole Acetate anstelle von PEG-5-Phytosterol formuliert werden.

### Beispiel 10

| Liposomenhaltiges Gel | |
|---|---|
| Lecithin | 6,00 |
| Pflanzliches Öl | 12,50 |
| Hydrolysiertes Kollagen | 2,00 |
| Xanthan Gum | 1,40 |
| Butylenglycol | 3,00 |
| Lanosterol | 1,00 |
| Parfum, Konservierungsstoffe | ................... q.s. ................... |
| Wasser, VES | .............. ad 100,00 .............. |

Das o.g. Beispiel kann auch mit einer identischen Menge an Wollwachsalkohol, Cholesteryl Isostearat oder Glyceryllanolat anstelle von Lanosterol formuliert werden.

### Beispiel 11

| Duschpräparat mit Rückfetter | |
|---|---|
| Cocoamidodiacetat | 10,00 |
| Natriumlaurylsulfat | 25,00 |
| Kalium Cocyl Hydrolysiertes Kollagen | 5,00 |
| Macadamianußöl | 5,00 |
| Natriumchlorid | 0,60 |
| Disodium Sitostereth-14-Sulfosuccinat | 4,00 |
| Parfum, Konservierungsstoffe | ................... q.s. ................... |
| Wasser, VES | .............. ad 100,00 .............. |

Das o.g. Beispiel kann auch mit einer identischen Menge an PPG-12-PEG-50-Lanolin, Cholesteryl Hydroxystearat oder Dihydrocholeth-15 anstelle von Disodium Sitostereth-14-Sulfosuccinat formuliert werden.

### Beispiel 12

| Seifenstück | |
|---|---|
| Na-Salz aus Talgfettsäuren | 60,00 |
| Na-Salz aus Kokosöl | 28,00 |
| Natriumchlorid | 0,50 |
| Wollwachsalkohol | 5,00 |
| Parfum, Konservierungsstoffe | ................... q.s. ................... |
| Wasser, VES | .............. ad 100,00 .............. |

Das o.g. Beispiel kann auch mit einer identischen Menge an Glyceryllanolat, Lanosterol oder Soya Sterol anstelle von Wollwachsalkohol formuliert werden.

### Beispiel 13

| Syndetseife | |
|---|---|
| Natriumlaurylsulfat | 30,00 |
| Natriumsulfosuccinat | 10,00 |
| Kaliumcocoyl hydrolysiertes Kollagen | 2,00 |
| Dimethicon Copolyol | 2,00 |
| Paraffin | 2,00 |
| Maisstärke | 10,00 |
| Talcum | 10,00 |
| Glycerin | 3,00 |
| Wollwachsalkohol | 5,00 |
| Parfum, Konservierungsstoffe | ................... q.s. ................... |
| Wasser, VES | .............. ad 100,00 .............. |

Das o.g. Beispiel kann auch mit einer identischen Menge an Glyceryllanolat, Lanosterol oder Soya Sterol anstelle von Wollwachsalkohol formuliert werden.

### Beispiel 14

| Haarpflegemittel | |
|---|---|
| TEA-Cocoyl hydrolysiertes Kollagen | 30,00 |
| Monoethanolaminlaurylsulfat | 25,00 |
| Mandelöl | 2,00 |
| Natriumchlorid | 1,00 |
| Cholecalciferol | 2,50 |
| Parfum, Konservierungsstoffe | ................... q.s. ................... |
| Wasser, VES . | ............. ad 100,00 .............. |

Das o.g. Beispiel kann auch mit einer identischen Menge an β-Sitosterol, Cholesterol oder Stearyl Glycyrrhetinat anstelle von Cholecalciferol formuliert werden.

### Beispiel 15

| Pflegeshampoo | |
|---|---|
| Dinatriumlaurylsulfosuccinat | 6,00 |
| Cocoamidopropylbetain | 10,00 |
| Glycoldistearat | 5,00 |
| 7-Dehydrocholesterol | 2,00 |
| Parfum, Konservierungsstoffe | ................... q.s. ................... |
| Wasser, VES | .............. ad 100,00 .............. |

Das o.g. Beispiel kann auch mit einer identischen Menge an Lanosterol, Glyceryllanolat oder Stearyl Glycyrrhetinat anstelle von 7-Dehydrocholesterol formuliert werden.

### Beispiel 16

| Haarkur | |
|---|---|
| Cetylalkohol | 5,00 |
| Caprylic/Capric Triglyceride | 3,00 |
| Petrolatum | 2,00 |
| Wollwuachsalkohol | 0,50 |
| Glyceryllanolat | 3,50 |
| Parfum, Konservierungsstoffe | ................... q.s. ................... |
| Wasser, VES | .............. ad 100,00 .............. |

Das o.g. Beispiel kann auch mit einer identischen Menge an Cholesteryl Iso-stearat, Cholesteryloleat oder Dihydrolanosterol anstelle von Glyceryllanolat formuliert werden.

### Beispiel 17

| Haarspülung | |
|---|---|
| Cocoamidopropylbetain | 5,00 |
| Cetylalkohol | 2,00 |
| Propylenglycol | 2,00 |
| Citronensäure | 0,30 |
| Glyceryllanolat | 5,00 |
| Parfum, Konsetuierungsstoffe | ................... q.s. ................... |
| Wasser, VES | .............. ad 100,00 .............. |

Das o.g. Beispiel kann auch mit einer identischen Menge an Soya Sterol, Cholesterylhydroxystearat oder Laneth-10 anstelle von Glyceryllanolat formuliert werden.

### Beispiel 18

| Haarfestiger | |
|---|---|
| Polyvinylpyrrolidon/Vinylacetat/Vinylpropionat-Copolymer | 5,00 |
| Ethanol | 45,00 |
| β-Sitosterol | 2,00 |
| Parfum, Konservierungsstoffe | ................... q.s. ................... |
| Wasser, VES | .............. ad 100,00 .............. |

Das o.g. Beispiel kann auch mit einer identischen Menge an Ergocalciferol, Laneth-15 oder PPG-5-Lanolin Wax anstelle von β-Sitosterol formuliert werden.

### Beispiel 19

| Frisiercrème | |
|---|---|
| Vaseline | 4,00 |
| Cetearylalkohol | 4,00 |
| PEG-40-hydriertes Rizinusöl | 2,00 |
| Isopropylpalmitat | 5,00 |
| Citronensäure | 1,00 |
| 7-Dehydrocholesterol | 2,00 |
| Parfum, Konservierungsstoffe | ................... q.s. ................... |
| Wasser, VES | .............. ad 100,00 .............. |

Das o.g. Beispiel kann auch mit einer identischen Menge an Cholesterol, Wollwachsalkohol oder Soya Sterol anstelle von 7-Dehydrocholesterol formuliert werden.

### Beispiel 20

| Rasierschaum | |
|---|---|
| Stearinsäure | 7,00 |
| Natriumlaurylsulfat | 3,00 |
| Stearylalkohol | 1,00 |
| Glycerin | 5,00 |
| Triethanolamin | 3,60 |
| Soya Sterol | 1,00 |
| Parfum, Konservierungsstoffe | ................... q.s. ................... |
| Wasser, VES | .............. ad 100,00 .............. |

Das o.g. Beispiel kann auch mit einer identischen Menge an Cholesterol, Wollwachsalkohol oder Glyceryllanolat anstelle von Soya Sterol formuliert werden.

### Beispiel 21

| Fußcrème | |
|---|---|
| Soluan 5 | 2,00 |
| Methylsalicylat | 5,00 |
| Caprylic/Capric Triglyceride | 10,00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| Wollwachsalkohol | 1,50 |
| Parfum, Konservierungsstoffe | ................... q.s. ................... |
| Wasser, VES | .............. ad 100,00 .............. |

Das o.g. Beispiel kann auch mit einer identischen Menge an Dihydrocholesterol, Dihydrolanosterol oder Soya Sterol anstelle von Wollwachsalkohol formuliert werden.

### Beispiel 22

| Aerosolspray | |
|---|---|
| Octyldodecanol | 0,50 |
| 7-Dehydrocholesterol | 1,50 |
| Parfum, Konservierungsstoffe | ................... q.s. ................... |
| Ethanol | .............. ad 100,00 .............. |

Das o.g. Beispiel kann auch mit einer identischen Menge an Wollwachsalkohol, Glyceryllanolat oder Soya Sterol anstelle von 7-Dehydrocholesterol formuliert werden.

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 23

| Pumpspray | |
|---|---|
| PEG-40-Hydriertes Rizinusöl | 2,00 |
| Glycerin | 1,00 |
| Dihydrophytosteryl Octyldecanoat | 2,00 |
| Parfum, Konservierungsstoffe | ................... q.s. ................... |
| Wasser, VES | .............. ad 100,00 .............. |

Das o.g. Beispiel kann auch mit einer identischen Menge an 7-Dehydrocholesterol, Cholesteryloleat oder Cholesteryl Isostearat anstelle von Dihydrophytosteryl Octyldecanoat formuliert werden.

### Beispiel 24

| Roll-on-Gel | |
|---|---|
| 1,3-Butylenglycol | 2,00 |
| PEG-40-Hydriertes Rizinusöl | 2,00 |
| Hydroryethylcellulose | 0,50 |
| Dihydrolanosterol | 2,50 |
| Parfum, Konservierungsstoffe | ................... q.s. ................... |
| Wasser, VES | .............. ad 100,00 .............. |

Das o.g. Beispiel kann auch mit einer identischen Menge an Glyceryllanolat, Soya Sterol oder Dihydrocholeth-20 anstelle von Dihydrolanosterol formuliert werden.

### Beispiel 25

| Roll-on-Emulsion | |
|---|---|
| Tricetearethphosphat | 0,30 |
| Octyldodecanol | 2,00 |
| C₁₂₋₁₅-Alkylbenzoat | 2,00 |
| C₁₀₋₃₀-Alkylacrylat | 0,15 |
| Dihydrocholeth-20 | 4,00 |
| Parfum, Konservierungsstoffe | ................... q.s. ................... |
| Wasser, VES | .............. ad 100,00 .............. |

Das o.g. Beispiel kann auch mit einer identischen Menge an Laneth-15, PPG-40-PEG-60-Lanolin Oil oder Tall Oil Sterol anstelle von Dihydrocholeth-20 formuliert werden.

### Beispiel 26

| Wachsstift | |
|---|---|
| Hydriertes Rizinusöl | 5,00 |
| Bienenwachs | 6,00 |
| Ceresin | 30,00 |
| C₁₂₋₁₅-Alkylbenzoat | 17,00 |
| Wollwachsalkohol | 7,50 |
| Parfum, Konservierungsstoffe | ................... q.s. ................... |
| Octyldodecanol | .............. ad 100,00 .............. |

Das o.g. Beispiel kann auch mit einer identischen Menge an β-Sitosterol, Cholesterol oder Glyceryllanolat anstelle von Wollwachsalkohol formuliert werden.

### Beispiel 27

| Aerosolspray | |
|---|---|
| Octyldodecanol | 0,50 g |
| 7-Dehydrocholesterol | 0,50 g |
| Parfum, Konservierungsstoffe | ........................... q.s. .......................... |
| Ethanol | ...................... ad 100,00 .......................... |

Das o.g. Beispiel kann auch mit einer identischen Menge an Soya Sterol, Cholesteryloleat oder Cholesterylpalmitoleat anstelle von 7-Dehydrocholesterol formuliert werden.

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 28

| Pumpspray | |
|---|---|
| PEG-40-Hydriertes Rizinusöl | 2,00 |
| Glycerin | 1,00 |
| Glyceryllanolat | 2,50 |
| Parfum, Konservierungsstoffe | ................... q.s. .................. |
| Wasser, VES | ................... ad. 100,00 ......... |

Das o.g. Beispiel kann auch mit einer identischen Menge an PEG-25 Soya Sterol, Dihydrolanosterol oder Wollwachsalkohol anstelle von Glyceryllanolat formuliert werden.

Die Zahlenangaben in den Beispielen sind Gewichtsprozente.

Die erfindungsgemäßen Wirkstoffe können besonders vorteilhaft in Mikroemulsionen verwendet werden. Kosmetische und dermatologische Zubereitungen gemäß der Erfindung können besonders vorteilhaft als
a) unverdickte,
b) klassisch, z.B. durch Zusatz von Polyoxameren, Pluronics, Carragenanen oder Pflanzengummen verdickte,
c) durch Zusatz von A-B-A-Triblockcopolymeren (z.B. PEG-150-Distearat, Fa. Akzo Nobel) oder alpha, omega-bis-polyethoxylierte Silane oder Silikone) verdickte,
d) durch Zusatz von Sternpolymeren (z.B. PEG-300-Pentaerythrityl-tetrastearat oder hydrophob modifizierte Tetrakis-polyethoxylierte Silane und Silikone) verdickte,
e) durch Zusatz von A-B-A-B-Multiblock-Copolymeren, Starburst-Polymeren, Dendrimeren und anderen supramolekularen Vernetzern (z.B. Rheodol TWIS 399, Fa. KAO, oder PEG-120-Methylglucose-dioleat) verdickte Öl-in-Wasser- (O/W-), bikontinuierliche oder Wasser-in-Öl- (W/O-) Mikroemulsionen Verwendung finden.

### Beispiel 29

| | |
|---|---|
| 1,3-Di-(2-ethylhexyl)-cyclohexan | 35,00 |
| Glyceryllanolat | 5,00 |
| Sorbitanmonolaurat | 10,00 |
| Wasser (+ Zitronensäure bis pH 5,5) | 45,00 |

Das o.g. Beispiel kann auch mit einer identischen Menge an Dihydrolanosterol, Dihydrophytosteryl Octyldecanoat oder Cholesteryloleat anstelle von Glyceryllanolat formuliert werden.

### Beispiel 30

| | |
|---|---|
| 1,3-Di-(2-ethylhexyl)-cylcohexan | 33,00 |
| Laneth-10-Acetat | 5,00 |
| Sorbitanmonolaurat | 10,00 |
| Wasser (+ Zitronensäure bis pH 5,5) | 45,00 |
| PEG-150-Distearat | 2,00 |

Das o.g. Beispiel kann auch mit einer identischen Menge an Choleth-20, Laneth-5 oder PPG-10-Lanolin Alcohol Ether anstelle von Sorbitanmonolaurat formuliert werden.

### Beispiel 31

| | |
|---|---|
| Steareth-15 | 4,80 |
| Glycerin-monostearat | 2,40 |
| Glyceryllanolat | 2,50 |
| Cyclomethicon | 3,30 |
| Cetearyloctanoat | 1,70 |
| Wasser | 85,30 |

Das o.g. Beispiel kann auch mit einer identischen Menge an Cholesteryl Iso-stearat, Glycyrrhetinyl Stearat oder Soya Sterol Acetate anstelle von Glyceryllanolat formuliert werden.

### Beispiel 32

| | |
|---|---|
| Steareth-15 | 4,80 |
| Glycerin-monostearat | 2,40 |
| Cholesteryl Hydroxystearat | 2,50 |
| Cyclomethicon | 3,30 |
| Cetearyloctampat | 17,00 |
| Wasser | 83,30 |
| PEG-150-Distearat | 2,00 |

Das o.g. Beispiel kann auch mit einer identischen Menge an β-Sitosteryl Acetat, Cholesteryl Oleat oder Dihydrophytosteryl Octyldecanoat anstelle von Cholesteryl Hydroxystearat formuliert werden.

## Patentansprüche

1. Verwendung einer Verbindung oder mehrerer Verbindungen aus der Gruppe der Sterole und Sterolderivate als antiadhäsive Wirkstoffe gegenüber grampositiven Bakterien in Desodorantien.

2. Verwendungen oder Zubereitungen gemäß Anspruch 1, **dadurch gekennzeichnet, daß** die Sterole und Sterolderivate gewählt werden aus der Gruppe Cholesterol, 7-Dehydrocheolesterol, Dihydrocholesterol, Lanosterol, Dihydrolanosterol, Spongosterol, Koprosterol, Phytosterole, Cholesteryloleat und Cholesterylsulfat.

## Claims

1. Use of a compound or a number of compounds from the group consisting of the sterols and sterol derivatives as anti-adhesive active compounds against gram-positive bacteria in deodorants.

2. Uses or preparations according to Claim 1, **characterized in that** the sterols or sterol derivatives are chosen from the group consisting of cholesterol, 7-dehydro-cheolesterol [sic], dihydrocholesterol, lanosterol, dihydrolanosterol, spongosterol, coprosterol, phytosterols, cholesteryl oleate and cholesteryl sulphate.

## Revendications

1. Utilisation d'un composé ou de plusieurs composés choisis dans le groupe des stérols et dérivés de stérols, en tant que substances actives antiadhésives pour des bactéries à Gram positif dans des déodorants.

2. Utilisations ou préparations selon la revendication 1, **caractérisées en ce que** les stérols et dérivés de stérols sont choisis dans le groupe constitué par le cholestérol, le 7-déhydrocholestérol, le dihydrocholestérol, le lanostérol, le dihydrolanostérol, le spongostérol, le coprostérol, les phytostérols, l'oléate de cholestéryle et le sulfate de cholestéryle.
